Europäisches Patentamt

European Patent Office

Office européen des brevets

(19)

(11) Publication number: **0 222 923**
**A1**

# EUROPEAN PATENT APPLICATION

published in accordance with Art. 158(3) EPC

(21) Application number: 86903550.1

(22) Date of filing: 15.05.86

Data of the international application taken as a basis:

(86) International application number:
**PCT/JP 86/00247**

(87) International publication number:
**WO 86/06636 (20.11.86 86/25)**

(51) Int. Cl.⁴: **A 61 K 39/395,** C 07 K 15/04,
C 12 N 15/00, C 12 Q 1/00,
G 01 N 33/573, G 01 N 33/577

(30) Priority: 17.05.85 JP 106573/85

(43) Date of publication of application: 27.05.87
**Bulletin 87/22**

(84) Designated Contracting States: **DE FR GB IT**

(71) Applicant: Dainippon Pharmaceutical Co., Ltd., 25,
Doshomachi 3-chome Higashi-ku, Osaka-shi,
Osaka 541 (JP)
Applicant: SHIRAIMATSU SHINYAKU COMPANY LTD.,
37-1, Aza-Inaba, O'aza-Ukawa, Minakuchi-cho
Koka-gun, Shiga-ken (JP)

(72) Inventor: KUROOKA, Shigeru, 10-7, Nonaka 3-chome,
Fujiidera-shi Osaka 583 (JP)
Inventor: HIROISHI, Shingo, 37-2, Aza-Inaba,
Ooaza-Ukawa Minakuchi-cho, Kouka-gun
Shiga 529-17 (JP)
Inventor: MATSUYAMA, Shigeyuki, 1767,
Kita-tsuchiyama Tsuchiyama-cho, Kouka-gun,
Shiga 528-02 (JP)
Inventor: KANEKO, Toshiaki, 37-2, Aza-Inaba,
Ooaza-Ukawa Minakuchi-cho, Kouka-gun
Shiga 529-17 (JP)
Inventor: SUNAHARA, Noriyuki, 86-2,
Kawashimaarisugawa-cho Nishikyo-ku, Kyoto-shi
Kyoto 615 (JP)

(74) Representative: Perry, Robert Edward et al, GILL
JENNINGS & EVERY 53-64 Chancery Lane, London
WC2A 1HN (GB)

(54) **MONOCLONAL ANTIBODY.**

(57) An anti-human pancreatic α-amylase monoclonal antibody having the following properties: (a) capable of recognizing human pancreatic α-amylase; (b) substantially unable to recognize human salivary α-amylase; and (c) not deactivating human pancreatic α-amylase even after its immunoreaction with said enzyme as the antigen. A method for assaying human pancreatic α-amylase using the monoclonal antibody, a method for separating human pancreatic α-amylase from human salivary α-amylase, and a reagent therefor are also disclosed. Separation and assaying of human pancreatic α-amylase and human salivary α-amylase in human body liquid such as blood serum serve to diagnose various diseases, particularly pancreatic diseases.

SPECIFICATION

MONOCLONAL ANTIBODY

Technical Field

This invention relates to an anti human pancreatic
α-amylase monoclonal antibody (hereinafter referred to as
"anti P-Amy monoclonal antibody"), a quantitative determination of a human pancreatic α-amylase (hereinafter referred
to as "P-Amy"), and a method for separation of P-Amy and a
human salivary glands α-amylase (hereinafter referred to as
"S-Amy") and a reagent useful for the separation.

Technical Background

It is useful to know the increase or decrease of α-
amylase in human body fluids such as serum for the purpose
of diagnosis of various diseases, particularly pancreatic
diseases, and therefore, the measurement of α-amylase is one
of the important items which are frequently used in clinical
tests. Human α-amylase includes two kinds of isozymes, i.e.
P-Amy and S-Amy, and correct diagnosis is difficult by
merely measuring total α-amylase activity, and hence, the
above two isozymes are usually subjected to differential
quantitative determination. Conventional differential
quantitative determination methods are, for example,
electrophoretic method and inhibitor method.

The electrophoretic method comprises applying the test samples to various carriers, subjecting to electrophoresis, staining the thus separated isozymes, and then
measuring color density to know the activity of P-Amy and S-
Amy. According to this method, however, the separation of
P-Amy and S-Amy can not completely be done and hence precise
differential determination can not be done. Moreover, this
method requires much longer time in the whole procedure and
hence is inconvenient for testing a large number of samples.

On the other hand, the inhibitor method is carried
out by using an S-Amy inhibitor of wheat origin, and the
ratio of the activity of P-Amy to that of S-Amy is calculated from difference of the degree of inhibition. However,
this method has low degree of specific inhibition to S-Amy
and hence precise determination can not be done. Moreover,
this method has much variation among different test lots,
and hence, a standard calibration curve must be drawn, which
is very troublesome.

Thus, the conventional methods are not satisfactory
in view of various requirements such as speediness and
precision quantitative determination, and the like.

It is known that P-Amy and S-Amy have very similar
amino acid sequence each other and that the homology reaches
94 % [cf. Gene, 28, 263-270 (1984)]. That is, the difference in the amino acid sequence between them is merely 6 %.
Accordingly, a monoclonal antibody against the P-Amy or S-
Amy must be able to recoginize such a few difference of each antigen. Besides, when there exists an anti S-Amy monoclonal antibody which binds specifically to S-Amy, the
epitope (antigen-determining group) to be recognized by the
anti P-Amy monoclonal antibody must be elected from such
narrower scope. Thus, it is very difficult to obtain anti
P-Amy monoclonal antibody which can bind specifically to P-
Amy. ,

Furthermore, S-Amy is commercially available, but
P-Amy is not commercially available, and hence, it is not
easier to get P-Amy in comparison with S-Amy. It has also
been found by the present inventors that even when human
pancreatic juice per se is administered to an animal
together with Freund's complete adjuvant, the animal can not
enough be immunized. These facts show that the production
of anti P-Amy monoclonal antibody is difficult.

It has been reported that the differention of P-Amy
and S-Amy was done by using anti S-Amy monoclonal antibody
[cf. J. Biochem., 97 (5), 1357-1362 (1985)], but there is
therein merely disclosed anti S-Amy monoclonal antibody, and
it is mentioned that they failed in the cloning of anti P-
Amy monoclonal antibody-producing cells.

Disclosure of the Invention

An object of this invention is to provide anti P-
Amy monoclonal antibody which has a high specificity and is
useful for the differential quantitative determination of P-
Amy and S-Amy.

Another object of this invention is to provide a method for the quantitative determination of P-Amy and a method for the differential quantitative determination of P-Amy and S-Amy.

A further object of this invention is to provide a method for the separation of P-Amy and S-Amy and also a kit useful therefor.

These and other objects of this invention will be apparent from the following description.

This invention relates to an anti P-Amy monoclonal antibody having the following characteristics:

(a) It recognizes P-Amy.

(b) It does substantially not recognize S-Amy.

(c) Even after it is subjected to immuno-reaction with an antigen P-Amy, the said P-Amy does not lose the enzymatic activity .

The monoclonal antibody of this invention can be prepared by cell fusion method. More particularly, the monoclonal antibody of this invention can be prepared by the following steps (1) to (4):

(1) steps of preparing an antigen and B cells,

(2) steps of fusion, screening and cloning,

(3) a step of culturing hybridoma, and

(4) a step of purification which is optionally carried out.

Each step is explained in more detail below.

(1) Steps of preparing an antigen and B cells:

B cells are collected from the spleen of an animal which is well immunized with an antigen P-Amy.

The antigen P-Amy is most preferably highly purified and is used in a large amount, but P-Amy having not so high purity may be used. For instance, a partially purified human pancreatic juice is used as an antigen. A partial purification of P-Amy from human pancreatic juice can be done, for instance, by fractionation with ammonium sulfate.

Immunization of an animal can be carried out by administering the antigen P-Amy to a mammal such as mouse or rat. Conditions for immunization, such as amount of the antigen P-Amy, injection site, kinds and amount of the adjuvant, etc. may be the same as those conventionally used for production of antiserum. The immunization is usually carried out by administering parenterally an emulsion containing P-Amy and Freund's complete adjuvant to a mammal (e.g. mouse), followed by additional immunization in the same manner as above at an interval of about one week. The additional immunization is usually carried out 4 to 7 times. Three to five days after the final immunization, the spleen is taken out from the mammal and B cells are collected therefrom.

(2) Steps of fusion, screening and cloning:

The fusion is carried out by suspending the above B cells and known myeloma cells in a known serum medium for cell fusion in the presence of a fusion accelerator and mixing them.

The myloma cells are preferably originated from the same animal as that used for immunization in the above step (1) and do not grow in a medium used for selection of hybridoma and do not secrete any antibody by themselves. Such myeloma cells include, for example, a commercially available mouse myeloma cell line P3-X63-Ag8-Ul or equivalents thereof.

Both cells are usually used in a ratio of 1 to 20 of B cells to 1 of myeloma cells. The fusion accelerator includes, for example, polyethylene glycol which has preferably a molecular weight of 1,000 to 7,500.

The cultivation of fused cells, i.e. hybridoma, is carried out by washing out the fusion accelerator therefrom, putting 0.1 - 0.2 ml of a suspension of the hybridoma in a medium for myeloma cells into each well on a 96-well titer plate (hereinafter optionally referred to merely as "well") and then incubating at about 37°C in 5 % carbon dioxide - air. During the cultivation, a known medium for selection of hybridoma such as HAT medium is added to the medium with gradually increasing the addition ratio of the medium. By such an exchange of medium, cells other than hybridoma die. Finally, the medium for selection of hybridoma is exchanged with a medium for myeloma cells, i.e. a medium for hybridoma.

The screening of the desired hybridoma can be carried out by checking the antibody titer and degree of cross reaction. That is, P-Amy or S-Amy and an insolubilized second antibody as mentioned hereinafter are added to a part of the culture medium, and the mixture is incubated, centrifuged, and then the enzymatic activity of the resulting pellet is measured by a method as mentioned hereinafter, by which the antibody titer and the degree of cross reaction of P-Amy and S-Amy can be measured.

The screened hybridoma is cloned by a limited dilution method and thereby there can be obtained the desired hybridoma which can substantially recognize only P-Amy. The hybridoma thus obtained can semi-permanently be kept by successive culture or in frozen state.

(3) Step of culturing hybridoma:

The hybridoma obtained in the above step is cultured _in vitro_ or _in vivo_, by which the desired monoclonal antibody can be obtained.

The _in vitro_ cultivation can be carried out by firstly cultivating several hybridoma on the 96-well titer plate, followed by cultivating with gradually increasing the scale. The _in vivo_ cultivation can be carried out by intraperitoneal inoculation of the hybridoma to a mouse which is previously treated with pristane in order to make the growth of the fused cells easier. Ten to 20 days after the inoculation, a peritoneal fluid containing the desired monoclonal antibody is accumulated.

Generally, the in vitro cultivation is done for producing a monoclonal antibody having high purity, and the in vivo cultivation is done in case of producing the monoclonal antibody in a large amount. By the in vivo cultivation, the anti P-Amy monoclonal antibody can usually be obtained in an amount of 10 to 100 mg per one mouse. For immunological analysis, the peritoneal fluid obtained by the in vivo cultivation can be used as it stands, but it may be further purified when necessary.

(4)  Step of purification:

The separation and purification of monoclonal antibody from the in vitro culture medium or from the in vivo culture peritoneal fluid is optionally carried out by conventional physicochemical methods, for example, by an appropriate combination of salting out, centrifugation, dialysis, various column chromatographys such as affinity chromatography using protein A bound to an insoluble carrier, and the like.

The anti P-Amy monoclonal antibody thus obtained can differentiate P-Amy and S-Amy. Besides, even after reacting immunologically with the monoclonal antibody of this invention, the P-Amy can decompose various kinds of substrates having various molecular weights, such as p-nitrophenylmaltoheptoside (hereinafter referred to as "PNP") (Boehringer Mannheim GmbH) which has a low molecular weight, or an insoluble blue starch polymer (Daiichi Kagaku Yakuhin K.K.) which has a high molecular weight.

The monoclonal antibody of this invention is useful as a reagent for differential quantitative determination of P-Amy and S-Amy.

The quantitative determination of P-Amy can be done by the procedure comprising the following steps:

(i)  a step of adding the monoclonal antibody of this invention to a test sample and incubating the mixture (immuno-reaction step),

(ii)  a step of separation of the produced antigen-antibody complex from other materials (usually by centrifugation) (step of separation of antigen-antibody complex), and

(iii)  a step of measuring the activity of α-amylase in the separated antigen-antibody complex (usually precipitate) (step of assay for α-amylase).

Thus, according to the method of this invention, the activity of P-Amy can directly be measured.

The differential quantitative determination of P-Amy and S-Amy can be done by measuring the activity of α-amylase in the antigen-antibody complex and other materials (usually supernatant) as separated in the above-mentioned step (ii), or alternatively, by subtracting the activity of P-Amy from the activity of total α-amylase which is independently measured.

The steps (i) to (iii) for the quantitative determination of P-Amy· is carried out at about 37°C. The anti P-Amy monoclonal antibody in the step (i) is usually used in an excess amount over the amount corresponding to P-Amy contained in the test sample. The step (ii) can be carried out by a technique used for B/F separation in enzyme immunoassay. For instance, an insolubilized anti P-Amy monoclonal antibody is used instead of the anti P-Amy monoclonal antibody in the step (i), and after the immuno- reaction, the antigen-antibody complex is separated by centrifugation, or an insolubilized antibody against the antigen-antibody complex, i.e. an insolubilized second antibody, is further reacted in the step (ii) and the resulting complex is separated by centrifugation. The insolubilization of antibody can be done by binding the anti P-Amy monoclonal antibody or the second antibody to an insoluble carrier by a conventional method. The insoluble carrier includes all conventional carriers, preferably bacterial cell wall debris as disclosed in U.S. Patent 4,166,767. The step (iii) and the measurement of total α- amylase can be done by known methods, for example by using a commercially available kit for quantitative determination of α-amylase. The kit includes Autopack α-amylase kit (sold by Boehringer Mannheim - Yamanouchi). In this kit, a solution containing PNP and α-glucosidase (hereinafter referred to as "PNP reagent") is used as a substrate.

The reagent used for the separation of P-Amy and S-Amy can be used in combinations with any commercially available kits for the quantitative determination of α-amylase. The reagent of this invention used for the separation of P-Amy and S-Amy contains at least

(1) an anti P-Amy monoclonal antibody bound to an insoluble carrier, or

(2) (a) an anti P-Amy monoclonal antibody and (b) a second antibody bound to an insoluble carrier (insolubilized second antibody).

The reagent may further contain buffering agents, preservatives, stabilizing agents, additives, and the like. In case of the above (2), it is preferable to use in the form of a kit.

Best Mode for Practice of the Invention

This invention is illustrated by the following Examples, wherein the following media are used.

I: A serum-free medium for cell fusion: RPM1 1640 medium (Gibco Laboratory)

II: Medium for myeloma cell (or hybridoma): a medium of the above I supplmented with the following ingredients:

10 % fetal calf serum (FCS),

4 mM glutamine,

50 μM β-mercaptoethanol,

100 U/ml penicillin G, and

100 µg/ml streptomycin

III: HAT medium (a medium for selection of hybridoma): a medium of the above II supplemented with the following ingredients:

0.1 mM hypoxanthine,

0.4 µM aminopterine, and

16 µM thymidine

In the following description, the phosphate buffer and bovine serum albumin are abbreviated as PBS and BSA, respectively.

Example 1    Preparation of anti P-Amy monoclonal antibody

(1)  Establishment of hybridoma:

Preparation of antigen and cells

To human pancreatic juice (2 ml) is added ammonium sulfate (554 mg) at 4°C (45 % saturation), and the mixture is allowed to stand at 0°C for 3 hours. The resulting pellets are centrifuged (5000 x g, 30 minutes) and dissolved in physiological saline solution (0.5 ml).

To the above solution is added Freund's complete adjuvant (Nakarai Kagaku Yakuhin K.K.) and emulsified. The emulsion (0.5 ml) is intraperitoneally administered to BALB/c mouse (Shizuoka Jikken Dobutsu Kyodo Kumiai), and after one week, the animal is immunized again in the same way. Further after one week, additional immunization (intravenous injection) is carried out. Thereafter, additional immunizations are repeated 6 times likewise.
Three days after the final immunization, the spleen is taken
out and B cells are collected therefrom.

Screening and cloning

Mouse myeloma cells P3-X63-Ag8-U1 (ATCC catalogue
No. CRL 1597) (1 x $10^7$ cells) in logarithmic growth phase
and spleen cells (1 x $10^8$ cells) are mixed, and the mixture
is washed with the medium I by centrifugation (400 x g, 10
minutes) and thereto is added slowly (in one minute) 50 %
polyethylene glycol 1500 (Wako Junyaku Kogyo K.K.) (1 ml)
which is kept at 37°C, and the mixture is stirred slowly.
After one minute, the medium I (1 ml) which is kept at 37°C
is added to the mixture likewise. Thereafter, each one ml
of the medium I (20 ml) is added at an interval of 30
seconds, and the mixture is centrifuged (400 x g, 10
minutes) and the supernatant is removed. To the resulting
pellets is added gradually the medium II (12 ml).
Each 0.1 ml of the mixture is distributed into every well of
the 96-well titer plate and subjected to cultivation at 37°C
in 5 % carbon dioxide - air. After 20 hours, 0.1 ml of the
medium III is added to each well. On 2nd, 3rd, 5th, 8th,
11th, 13th and 15th days (7 times in total), the old medium
(0.1 ml) is removed, and a fresh medium III (0.1 ml) is
added. By the exchange of medium, cells other than
hybridoma die. After 16 days, the medium is again changed
to the medium II, and it is cultivated. Seven days after the start of the cultivation, the hybridoma starts to grow and on 15th day the growth thereof is observed in 80 % of the wells.

Determination of antibody titer and cross reaction

The antibody titer and cross reaction of the culture liquid after 15 days cultivation are determined as follows.

Reagent for determination

(1) P-Amy solution:

Pancreatic juice (250 µl, 92,000 IU/liter) is diluted with the following Buffer A to make a final volume of 50 ml.

(2) S-Amy solution:

S-Amy (Sigma Chemical Co., 1 mg) is dissolved in and diluted with the following Buffer A to make a final volume of 50 ml.

(3) Buffer A (pH 7.0):

A buffer solution consisting of 0.1 % BSA - 0.9 % NaCl - 0.04 M PBS (pH 7.0)

(4) Suspension of insolubilized second antibody:

It is prepared in the same manner as described in U.S. Patent 4,166,767. That is, cell wall debris (125 mg) of Lactobacillus plantarum ATCC 8014 is suspended in water (2.5 ml) and thereto are added anti-mouse IgG rabbit antibody solution [protein 25 mg/0.05 M PBS (pH 7.0) 2.5 ml] (manufactured by Mils-Yeda Ltd.), water (2.5 ml) and 0.1 M PBS (pH 7.0) (2.5 ml), and the mixture is well mixed. To the mixture is added dropwise 2.5 % glutaraldehyde solution (2.5 ml) with stirring. After stirring for one hour, the mixture is centrifuged (2,000 x g, 10 minutes), and the precipitate is washed three times with 0.9 % NaCl - 0.02 M Tris buffer (pH 7.0) (5 ml) to give the desired insolubilized second antibody. This product is suspended in 0.2 % BSA - 0.9 % NaCl - 0.1 % $NaN_3$ Tris buffer (pH 7.0) (5.2 ml) and is subjected to ultrasonication.

Procedure for determination

The antibody titer is determined in the following manner.

That is, the culture medium of hydridoma (50 µl), P-Amy solution (25 µl) and the suspension of insolubilized second antibody (50 µl) are mixed, and the mixture is incubated at 37°C for 30 minutes, and thereto is added physiological saline solution (4 ml). The mixture is centrifuged (3000 x g, 5 minutes), and the supernatant is removed. To the resulting pellets is added PNP reagent (1 ml), and the mixture is stirred and then incubated at 37°C. After 30 minutes, a reaction terminator solution [0.1 M PBS (pH 11), 2 ml] is added thereto, and the reaction mixture is centrifuged, and the absorbance (405 nm) of the supernatant is measured. The well giving a higher absorbance is evaluated to contain an anti P-Amy monoclonal antibody having a higher antibody titer.

The degree of cross reaction is investigated as to the culture medium having a high antibody titer.

That is, in the same manner as described in the above determination of antibody titer except that S-Amy solution is used instead of P-Amy solution, the degree of cross reaction is determined. The test sample having a lower absorbance at 405 nm is evaluated to have a lower cross reactivity.

A strain being capable of producing anti P-Amy monoclonal antibody having particularly high antibody titer and low cross reactivity is selected, and the hybridoma cells are diluted with the medium II so as to give a concentration of one cell per well on the 96-well titer plate and then cultivated. By repeating the above limited dilution, the cloning is achieved to give the desired hybridoma.

The hybridoma can be kept in frozen state or by successive culture.

(2) Preparation of monoclonal antibody:

BALB/c mouse which is previously intraperitoneally administered with pristane (0.5 ml) is intraperitoneally inoculated with the hybridoma (1 x $10^7$ cells) obtained as above. After 15 days, there is obtained a peritoneal fluid (3 ml/mouse) containing the desired monoclonal antibody. To the peritoneal fluid is added an equivolume of saturated ammonium sulfate solution, and the mixture is stirred at 4°C for 30 minutes and centrifuged (5000 x g, 30 minutes), and the supernatant is removed. The pellet is dissolved in 0.05 M PBS (pH 7.4) - 0.14 M sodium chloride solution. This solution is dialyzed at 4°C overnight, centrifuged (5000 x g, 30 minutes), and the resulting supernatant is lyophilized to give crude monoclonal antibody.

According to the determination based on an immuno-diffusion method, the monoclonal antibody belongs to IgG1 subclass and the L chain thereof belongs to kappa.

Example 2    Differential quantitative determination

A test sample is prepared by diluting a solution containing an equal amount of P-Amy and S-Amy by a two fold dilution method, and it is subjected to the differential quantitative determination in the following manner.

Quantitative determination of total α-amylase activity

To the test sample (25 μl) is added PNP reagent (1 ml), and the mixture is incubated at 37°C for 30 minutes, and thereafter, there is added 0.1 M PBS (pH 11) (2ml) (reaction terminator), and the absorbance of the reaction mixture at 405 nm is measured.

Quantitative determination of P-Amy

The test sample (25 μl), an anti P-Amy monoclonal antibody [which is prepared by diluting the peritoneal fluid obtained in Example 1-(2) in 1,000-fold with Buffer A] (50 μl), and a suspension of insolubilized second antibody (50 µl) are mixed, and the mixture is incubated at 37°C for 30 minutes, and to the reaction mixture is added physiological saline solution (4 ml), and the mixture is centrifuged (3000 x g, 5 minutes). To the resulting pellet is added PNP reagent (1 ml), and the mixture is incubated at 37°C for 30 minutes, and thereafter is added a reaction terminator (2 ml) and the insolubilized second antibody is removed by centrifugation (3000 x g, 5 minutes). The absorbance of the supernatant at 405 nm is measured in a cuvette (light path: 1 cm).

Calculation of S-Amy

The S-Amy activity is calculated by subtracting the P-Amy activity from total α-amylase activity.

Results of the quantitative determination

The results are shown in Table 1.

Table 1

| Dilution fold | P-Amy activity (a) (OD/ml) | S-Amy activity (b) (OD/ml) | b/a |
|---|---|---|---|
| 1 | 0.800 | 0.801 | 1.00 |
| 2 | 0.415 | 0.401 | 0.97 |
| 4 | 0.200 | 0.203 | 1.02 |
| 8 | 0.100 | 0.101 | 1.01 |
| 16 | 0.051 | 0.048 | 0.94 |
| 32 | 0.024 | 0.025 | 1.04 |
| 64 | 0.011 | 0.012 | 1.09 |

As is shown in Table 1, the ratio of P-Amy and S-Amy, i.e. b/a is always about 1.00, and the method of this invention is extremely excellent for the differential quantitative determination of both.

Example 3    Differential quantitative determination

P-Amy, S-Amy and mixtures of both (mixing ratios are shown in Table 2) are used as test samples, and they are subjected to the differential quantitative determination in the following manner to give the results as shown in Table 2.

Quantitative determination of total α-amylase activity

To the test sample (40 μl) is added Buffer A (150 μl), and the mixture is incubated at 37°C for 10 minutes. The reaction mixture (25 μl) is taken out, and thereto is added PNP reagent (1 ml), and the mixture is incubated at 37°C for 30 minutes, and thereto is added a reaction terminator (2 ml), and the absorbance of the reaction mixture at 405 nm is measured in a cuvette (light path: 1 cm).

### Quantitative determination of S-Amy

To the test sample (40 µl) is added a suspension (150 µl) of an insolubilized anti P-Amy monoclonal antibody which is prepared in the same manner as described in the item of Detection of antibody titer hereinbefore, and the mixture is incubated at 37°C for 10 minutes and then centrifuged (3000 x g, 5 minutes). The supernatant (25 µl) is taken out and thereto is added PNP reagent (1 ml), and the mixture is incubated at 37°C for 30 minutes, and thereafter is added a reaction terminator (2 ml), and the absorbance at 405 nm is measured in a cuvette (light path: 1 cm).

### Results of the quantitative determination

The results are shown in Table 2.

Table 2

| Test sample | Total α-amylase activity (a) (OD/ml) | Activity of[**] supernatant (b) (OD/ml) | b/a |
|---|---|---|---|
| (1) P-Amy | 0.165 | 0 | 0.00 |
| (2) S-Amy[*] | 0.372 | 0.375 | 1.01 |
| (3) S + P[*] | 0.550 | 0.370 | 0.67 |
| [S/(P+S) = 0.7] | | | |
| (4) P-Amy | 0.344 | 0 | 0.00 |
| (5) S-Amy | 0.330 | 0.332 | 1.01 |
| (6) S + P | 0.660 | 0.337 | 0.51 |
| [S/(P+S) = 0.5] | | | |
| (7) P-Amy | 0.413 | 0 | 0.00 |
| (8) S-Amy | 0.179 | 0.173 | 0.97 |
| (9) S + P | 0.619 | 0.180 | 0.29 |
| [S/(P+S) = 0.3] | | | |

*)    A mixture of P-Amy and S-Amy, wherein both are mixed so that the ratio [S/(P+S)] of S-Amy to total α-amylase (P + S) becomes 0.7.  Thereafter, the same.

**)   It corresponds to S-Amy activity.

The P-Amy in the test sample is removed from the system by the insolubilized anti P-Amy monoclonal antibody, and hence, as is shown in Table 2, in the test samples (1), (4) and (7) which contain only P-Amy, the (b) value (corresponding to S-Amy activity) is all approximately zero.  On the other hand, since S-Amy is not removed from the system, in the test samples (2), (5) and (8) which contain only S-Amy, the (b) value (corresponding to S-Amy activity) is approximately equal to the (a) value (total α-amylse activity).  Besides, as is shown in Table 2, the mixing ratio of P-Amy and S-Amy [P/(P+S)] is approximately consistent to the b/a ratio.  Thus, the method for differential quantitative determination of this invention is very excellent.

Example 4    Differential quantitative determination of human serum

Sera of patients with pancreatic disease and of healthy subjects were used as a test sample, and the quantitative determination of P-Amy was carried out in the same manner as described in Example 2.  The results are shown in Table 3.

Table 3

| Test sample | Total α-amylase activity (a) (OD/ml) | Activity of P-Amy (b) (OD/ml) | b/a |
|---|---|---|---|
| Serum of patients.: | | | |
| NO (pancreatitis) | 0.315 | 0.251 | 0.80 |
| GO (pancreatitis) | 0.960 | 0.803 | 0.84 |
| NI (pancreatitis) | 0.175 | 0.121 | 0.69 |
| SN (sialadenitis) | 0.870 | 0.023 | 0.03 |
| Serum of healthy subjects: | | | |
| FO | 0.175 | 0.055 | 0.31 |
| ON | 0.146 | 0.061 | 0.42 |
| TH | 0.130 | 0.041 | 0.32 |

As is shown in Table 3, the ratio (b/a) of P-Amy to total α-amylase in the sera of patients with pancreatitis (NO, GO, NI) was higher than that in healthy subjects, and that of patient with sialadenitis (SN) was lower.

- 23 -

Industrial Utility

The anti P-Amy monoclonal antibody of this invention is useful for the differential quantitative determination of P-Amy and S-Amy. When P-Amy and S-Amy in human body fluids such as serum is differentially determined, it can be used for diagnosis of various diseases, particularly pancreatic disease.

- 24 -

CLAIMS

(1)  An anti human pancreatic α-amylase monoclonal
antibody having the following characteristics:

(a)  it recognizes human pancreatic α-amylase,

(b)  it does substantially not recognize human
salivary glands α-amylase,

(c)  even after it is subjected to immuno-reaction
with an antigen human pancreatic α-amylase, the said human
pancreatic α-amylase does not lose the enzymatic activity.

(2)  The anti human pancreatic α-amylase monoclonal
antibody according to claim 1, to which an insoluble carrier
is bound.

(3)  The anti human pancreatic α-amylase monoclonal
antibody according to claim 2, wherein the insoluble carrier
is bacterial cell walls debris.

(4)  The anti human pancreatic α-amylase monoclonal
antibody according to claim 2 or 3, wherein the insoluble
carrier is cell wall debris of Lactobacillus plantarum

(5)  The anti human pancreatic α-amylase monoclonal
antibody according to claim 1, which is prepared by cell
fusion technique.

(6)  A method for the quantitative determination of
a human pancreatic α-amylase in a test sample, which
comprises (i) adding the anti human pancreatic α-amylase
monoclonal antibody as set forth in claim 1 to the test sample, and thereby subjecting to an immuno-reaction, (ii) separating the resulting antigen-antibody complex, (iii) measuring an activity of the α-amylase in the antigen-antibody complex.

(7) The method for the quantitative determination according to claim 6, wherein an anti human pancreatic α-amylase monoclonal antibody bound to an insoluble carrier is used, and the separation of the antigen-antibody complex is carried out by centrifugation.

(8) The method for the quantitative determination according to claim 6, wherein an antibody against the anti human pancreatic α-amylase monoclonal antibody, i.e. a second antibody, is applied to the immuno-reaction system, and the resulting complex is separated by centrifugation.

(9) The method for the quantitative determination according to claim 8, wherein a second antibody bound to an insoluble carrier is used.

(10) The method for the quantitative determination according to claim 7 or 9, wherein the insoluble carrier is bacterial cell walls debris.

(11) A method for the separation of human pancreatic α-amylase and human salivary glands α-amylase, which comprises (i) adding the anti human pancreatic α-amylase monoclonal antibody as set forth in claim 1 to a test sample and thereby subjecting to an immuno-reaction, and (ii) separating the resulting antigen-antibody complex.

(12) The method for the separation according to claim 11, wherein an antibody against an anti human pancreatic α-amylase monoclonal antibody, i.e. a second antibody, is applied to the immuno-reaction system, and the resulting complex is separated by centrifugation.

(13) The method for the separation according to claim 12, wherein a second antibody bound to an insoluble carrier is used.

(14) A reagent for the separation of human pancreatic α-amylase and human salivary glands α-amylase, which contains at least an anti human pancreatic α-amylase monoclonal antibody bound to an insoluble carrier.

(15) A reagent for the separation of human pancreatic α-amylase and human salivary glands α-amylase, which contains at least (a) an anti human pancreatic α-amylase monoclonal antibody and (b) a second antibody bound to an insoluble carrier.

(16) The reagent according to claim 15, which is in the form of a kit.

# INTERNATIONAL SEARCH REPORT

0222923

International Application No. PCT/JP86/00247

## I. CLASSIFICATION OF SUBJECT MATTER (if several classification symbols apply, indicate all) [3]

According to International Patent Classification (IPC) or to both National Classification and IPC

Int.Cl[4] A61K39/395, C07K15/04, C12N15/00, C12Q1/00, G01N33/573, 33/577

## II. FIELDS SEARCHED

### Minimum Documentation Searched [4]

| Classification System | Classification Symbols |
|---|---|
| IPC | A61K39/395, C07K15/04, C12N15/00, C12Q1/00, G01N33/573, 33/577 |

### Documentation Searched other than Minimum Documentation to the Extent that such Documents are Included in the Fields Searched [5]

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| A | JP, A, 58-183098 (Wako Pure Chemical Industries, Ltd.), 26 October 1983 (26. 10. 83) (Family: none) | 1 - 16 |
| A | Journal of Biochemistry, Vol.97, No.5, (1985), K. Ito et al. [Preparation of human salivary α-amylase specific monoclonal antibody] p.1357-1362 | 1 - 16 |
| P | Chemical Abstracts, Vol.103, No.15, 14 October 1985 (14. 10. 85), See, Abstract No. 118550c, Clinical Chemistry, 1985, 31(8). 1283-1286 | 1 - 16 |
| P | Chemical Abstracts, Vol.103, No.13, 30 September 1985 (30. 09. 85), See, Abstract No.100716b, Clinical Chemistry, 1985, 31(8), 1286 - 1288 | 1 - 16 |

* Special categories of cited documents: [15]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

## IV. CERTIFICATION

| Date of the Actual Completion of the International Search [2] | Date of Mailing of this International Search Report [2] |
|---|---|
| July 31, 1986 (31. 07. 86) | August 18, 1986 (18. 08. 86) |

| International Searching Authority [1] | Signature of Authorized Officer [20] |
|---|---|
| Japanese Patent Office | |

Form PCT/ISA/210 (second sheet) (October 1981)